# EUROPEAN PATENT SPECIFICATION

(11) **EP 1 332 223 B1**
(45) Date of publication and mention of the grant of the patent: **29.08.2007**
(21) Application number: 01993704.4
(22) Date of filing: 07.11.2001
(51) Int. Cl.: C12Q 1/04, C12N 1/20, A01N 63/00

(54) **METHOD FOR SCREENING PROBIOTIC STRAINS OF THE GENUS BIFIDOBACTERIUM**
SCREENING-VERFAHREN FÜR PROBIOTISCHE STÄMME DER GATTUNG BIFIDOBACTERIUM
RECHERCHE SYSTEMATIQUE DE SOUCHES PROBIOTIQUES DE BIFIDOBACTERIES

(30) Priority: 08.11.2000 FI 20002445
(43) Date of publication of application: 06.08.2003
(73) Proprietor: Raisio Yhtymä Oyj, 21200 Raisio (FI); Bioferme OY, 20760 Piispanristi (FI)
(72) Inventor: SALMINEN, Seppo, 20100 Turku (FI); OUWEHAND, Arthur, 10210 Inga (FI); SALMINEN, Eeva, 20100 Turku (FI); ISOLAURI, Erika, 01900 Nurmijärvi (FI)
(74) Representative: Karvinen, Leena Maria
(86) International application number: PCT/FI2001/000969
(87) International publication number: WO 2002/038798

(56) References cited:
- ARTHUR C. OUWEHAND ET AL.: 'Adhesion of four bifidobacterium strains to human intestinal mucus from subjects in different age groups' FEMS MICROBIOLOGY LETTERS vol. 172, 1999, pages 61 - 64, XP002963188
- PIRKKA V. KIRJAVAINEN ET AL.: 'The ability of probiotic bacteria to bind to human intestinal mucus' FEMS MICROBIOLOGY LETTERS vol. 167, 1998, pages 185 - 189, XP002963189
- ARTHUR C. OUWEHAND ET AL.: 'Probiotics: mechanisms and establised effects' INTERNATIONAL DAIRY JOURNAL vol. 9, 1999, pages 43 - 52, XP002963190
- COLUM DUNNE ET AL.: 'Probiotics: from myth to reality. demonstration of functionality in animal models of disease and in human clinical trials' ANTONIE VAN LEEUWENHOEK vol. 76, 1999, pages 279 - 292, XP000929178
- KAILA KAILASAPATHY ET AL.: 'Survival and therapeutic potential of probiotic organisms with reference to lactobacillus acidophilus and bifidobacterium spp.' IMMUNOLOGY AND CELL BIOLOGY vol. 78, 2000, pages 80 - 88, XP002963191
- YOSHIMI BENNO ET AL.: 'Comparison of fecal microflora of elderly persons in rural and urban areas of Japan' APPLIED AND ENVIRONMENTAL MICROBIOLOGY vol. 55, no. 5, May 1999, pages 1100 - 1105, XP002965143
- DATABASE BIOSIS [Online] BERNET MARIE-FRANCOISE ET AL.: 'Adhesion of human bifidobacterial strains to cultured human intestinal epithelial cells and inhibition of enteropathogen-cell interactions', XP002963192 Database accession no. PREV199497090953 & APPLIED AND ENVIRONMENTAL MICROBIOLOGY vol. 59, no. 12, 1993, pages 4121 - 4128
- DATABASE BIOSIS [Online] HE FANG ET AL.: 'Adhesion of bifidobacterium spp. to human intestinal mucus', XP002963193 Database accession no. PREV200100201438 & MICROBIOLOGY AND IMMUNOLOGY vol. 45, no. 3, 2001, pages 259 - 262
- R. CRITTENDEN ET AL.: 'Adhesion of bifidobacteria to granular starch and its implications in probiotic technologies' APPLIED AND ENVIRONMENTAL MICROBIOLOGY vol. 67, no. 8, August 2001, pages 3469 - 3475, XP002963187

## Description

### Field of the invention

The present invention relates to a method for screening bacteria which are beneficial to human health, and to specific strains selected using the method. In specific, the invention provides a method for screening probiotic strains of bifidobacteria, which can be used in production of functional and clinical food products or dietary supplements.

### Background of the Invention

Probiotics are viable bacteria, or their inactivated cells or cell wall components, that have a beneficial effect on human health [Salminen et al. (1999) Trends in Food Science and Technology 10:107-110]. Probiotics are most commonly viable Lactobacillus preparations, but recently also bifidobacteria and other microbes have been shown to have probiotic properties.

The importance of human intestinal microflora for the well-being of the human host, and the importance of the development of intestinal microflora after birth, during childhood, during adult ages and during the old age is in the background of the present invention.

At birth the newborn is practically sterile and is colonised by the microbes present in the birth channel of the mother and the environment. Bifidobacteria soon colonise a healthy infant and can be detected in the faeces from the 2^{nd} or 3^{rd} day after birth. The growth of bifidobacteria is promoted by breast feeding.

Besides bifidobacteria, lactobacilli are among the first colonisers of the intestinal tract following the birth of a human being. These two genera soon form the major basis of the intestinal microflora in health, preventing diseases in the early childhood. During growth and ageing the microflora becomes more diverse, but the role of lactobacilli and bifidobacteria remains important. Especially in Japanese studies it has been shown that bifidobacteria decrease and lactobacilli increase in numbers, and the diversity of strains present in the intestinal tract of the elderly are less than in adults or infants [Mitsuoka et al., (1974) Zbl. Bakt. Hyg. I Abt. Orig. A 266:469-478]. Consequently, it has been suggested that the *Lactobacillus* flora and the *Bifidobacterium* flora are important factors in promoting health and preventing or reducing the risk of disease. Supplementation of the microflora with selected strains from the genera *Lactobacillus* and *Bifidobacterium* may therefore improve the health and well-being of the host. The strains to be used for this purpose are to possess several advantageous properties in order to be potentially successful probiotic strains [Ouwehand et al. (1999), Int. Dairy J. 9:43-52].

The types of bifidobacteria present in the intestinal contents in the elderly and in infants vary, and healthy subjects have higher bifidobacterial numbers and different *Bifidobacterium* species. Especially bifidobacteria appear to bind less to the intestinal mucus of the elderly subjects but they may be more abundant in healthy very old subjects. Similarly, different strains of bifidobacteria colonise subjects from different age groups and patient populations.

On the other hand, there are specific strains of bifidobacteria that have documented health effects and these include prevention and treatment of diarrhoea in children and alleviation of food allergy symptoms in children.

### Summary of the invention

The present invention is based on the inventors' observation that bifidobacteria exhibit differential binding, not only on human intestinal mucus preparations of subjects from different age groups, but especially on whole human intestinal mucosa (*ex vivo*) of such groups.

On the basis of said observation a method for screening probiotic strains of bifidobacteria has been developed. The probiotic strains so obtained have desired properties and can be used in producing functional and clinical food products or dietary supplements.

The main object of the present invention is thus a method for screening probiotic strains of the genus *Bifidobacterium,* comprising isolating bifidobacteria from faecal specimens of healthy elderly human subjects, and by screening for their age- and disease-specific properties by screening for their adhesion to intestinal mucus glycoproteins (*in vitro*) and to whole human intestinal mucosa (*ex vivo*), screening for the ability of the strains to grow on cereal-based substrates and, optionally, further subjecting the bacteria to the specific selection steps as defined hereinafter, to obtain bacterial strains having specific probiotic properties.

Specific bifidobacterial strains obtained by the screening method are further objects of the invention. Such strains preferably show e.g. age-specific or disease-specific properties and, consequently, can be used in preparing functional foods for target populations, in specific, elderly people, or disease-specific functional foods for the reduction of risk of diseases, respectively.

### Detailed Description of the Invention

Our *in vitro* studies have shown that the adhesive properties of most bifidobacteria used commercially are lower in the elderly, and that in humans the types of bifidobacteria change in different age groups with decreasing adherence along with old age. Furthermore, our *ex vivo* studies with whole human intestinal mucosa, obtained from surgically removed colonic tissue, have shown similar adherence variation in different groups.

Consequently, besides showing that the types of bifidobacteria are specific for certain age groups, we have shown that adherence in the elderly can be used as a selection criterion for strains applicable to the intestinal conditions of the elderly.

We have isolated bifidobacteria from the faeces of healthy elderly human subjects and shown that these strains exhibit different binding properties *in vitro* and *ex vivo.* We have noticed that among the healthy and old individuals there is a population of bifidobacteria which shows good adherence on these subjects and thus increase the possibility of intestinal colonisation in such subjects with health-promoting effects on intestinal microflora and function.

It should also be noted that the screening method of the invention can also be applied to bacteria from faecal specimens from any appropriate animals, not only human beings.

In this specification the term "elderly", e.g. in the expression 'elderly human subjects' refers to individuals of about 60 years of age or older. On the other hand, the term "very old", e.g. in the connection of the expression 'very old human subjects' refers to individuals of about 80 years of age, or older.

The microbes are isolated from healthy individuals from freshly voided faecal specimens using specific media for isolating bifidobacteria. The isolated bacteria are selected following the described selection criteria and procedure.

*In vitro* adhesion of probiotics is generally assessed using enterocyte-like tissue culture cells or human intestinal mucus. The mucus used is usually isolated from fecal samples. Although these methods are well established, they take into account only one part of the intestinal mucosa; i.e. either the enterocytes or the overlying mucus layer. None of the currently used models takes into account the presence of the normal intestinal microflora.

The bifidobacteria described in this application have, in addition, been assessed for their adhesive ability to whole human intestinal mucosa, obtained from surgically removed colonic tissue. This new *ex vivo* model takes into account the interaction of the bifidobacteria with both the intestinal mucus and the enterocytes, and also the normal intestinal microflora. This provides more accurate knowledge on the adhesive abilities of the bifidobacteria than earlier models.

Consequently, specific bacteria are subjected to a test using whole human intestinal mucosa, and their age- and disease-dependent samples to select for specific bacteria adhering to the mucosa of very old healthy subjects. This screens for age-specific adhesion properties for the elderly. Whole mucosa from subjects with colon cancer, inflammatory bowel disease or other intestinal dysfunctions can be used to screen for disease-specific probiotic bifidobacteria.

In a preferred embodiment specific bacteria are subjected to a test using human intestinal mucus glycoproteins and their age- and disease-dependent samples to select for specific bacteria adhering to the mucus of very old healthy subjects. This also screens for age-specific properties for the elderly. Mucus from subjects with rotavirus diarrhoea or other intestinal dysfunctions can be used to screen for disease-specificity.

The screening for toxin binding is carried out by using mycotoxin and/or bacterial toxin binding as a model for intestinal toxin binding. Preferably the binding should be extensive, so that the adhering strains lose their adherence when toxin is bound.

Promising strains will be tested for their relative resistance to bile and low pH with the help of flow cytometry to select for strains that are likely to survive the passage through the intestinal tract.

The ability of the strains to grow on certain cereal-based substrates will be determined. Useful cereals are oat, rye, barley, wheat and rice. Mixtures of them or fractions of them can be used as well. Most preferred for the purposes of this invention is oat. Substrates of interest include cereal components like carbohydrates (starch, oligosaccharides, β-glucans) and protein or other fermentable components. Oat oligosaccharides or, in specific, oat fractions enriched in β-glucan, protein or starch are most preferable. This is important for functional food applications and health effects.

To allow for food technology and industrial processes, screening for relatively aerotolerant strains is needed. Said screening is effected by culturing the strains on blood liver agar plates in an atmosphere with reduced oxygen pressure.

Strains of interest will be subjected to competitive exclusion of model pathogens such as pathogenic *E. coli* strains, *Yersinia enterocolitica, Sabnonella typhimurium* or *S. enteritidis,* to select for the most effective strains that inhibit common intestinal pathogens.

Host-microbe interactions are tested for the influence of the host microflora on mucus binding properties of the selected strains. The aim is to use the specific target group's own microflora as a selection criterion for well adhering strains in age-specific and disease-specific populations.

Age- and disease-specificity are used as criteria for selecting strains. Disease-specific and age-specific intestinal segments received from surgical procedures are used for screening. The samples are used immediately following surgery. Specificity has been demonstrated for commercial strains in terms of age of the subjects and diseases.

A specific object of the present invention is thus a method for screening probiotic strains of the genus *Bifidobacterium,* comprising isolating bifidobacteria from faecal specimens of healthy elderly human subjects, and screening for their age- and disease-specific properties by screening for their adhesion to intestinal mucus glycoproteins and to whole intestinal mucosa, screening for the ability of the strains to grow on cereal-based substrates, as well as screening for the aerotolerance of the strains and, optionally, subjecting the bacteria to the steps of:
a) selecting for strains which, when binding intestinal toxins lose their adherence to the mucus,
b) selecting for strains likely to survive the passage through the intestinal tract, and
c) selecting for the most effective strains that inhibit common intestinal pathogens, and isolating and identifying the strains showing an appropriate selection of the above-indicated properties.

A specific set of *Bifidobacterium* strains has been characterised with such properties and will be described hereinafter. Some strains may indeed have these properties, while others will have only some of the mentioned properties. For instance, some strains bind aflatoxin best when boiled. Obviously, bile and acid resistance is not relevant for these organisms if they are used that way.

The following strains A to H, which form a further object of this invention, were isolated from faecal specimens from very old, healthy Japanese men. The strains were selected using the above-indicated selection criteria.

Strain A: *Bifidobacterium adolescentis* 5 shows good adhesion to resected colonic tissue. The strain also has high Aflatoxin B1 binding capacity both when viable and when heat-treated. This strain has been deposited according to the Budapest Treaty at Deutsche Sammlung von Mikroorganismen und Zellkulturen GmbH, Braunschweig, Germany, on October 24, 2000 with the accession number DSM 13797.

Strain B: *Bifidobacterium adolescentis* 15 shows good adhesion to resected colonic tissue and has high Aflatoxin B1 binding capacity both when viable and when heat-treated. This strain has been deposited according to the Budapest Treaty at Deutsche Sammlung von Mikroorganismen und Zellkulturen GmbH, Braunschweig, Germany, on October 24, 2000 with the accession number DSM 13798.

Strain C: *Bifidobacterium adolescentis* 19 has good adherence to intestinal mucus and good resistance to bile. It also has high Aflatoxin B 1 binding capacity both when viable and when heat-treated. This strain has been deposited according to the Budapest Treaty at Deutsche Sammlung von Mikroorganismen und Zellkulturen GmbH, Braunschweig, Germany, on October 24, 2000 with the accession number DSM 13799.

Strain D: *Bifidobacterium adolescentis* 34 has high Aflatoxin B1 binding capacity when heat-treated and has good acidification, growth and survival in oat-based media. This strain has been deposited according to the Budapest Treaty at Deutsche Sammlung von Mikroorganismen und Zellkulturen GmbH, Braunschweig, Germany, on October 24, 2000 with the accession number DSM 13800.

Strain E: *Bifidobacterium longum* 56 has high Aflatoxin B1 binding capacity after heat-treatment and exhibits some tolerance to low (2.5) pH. The strain also shows good adhesion to resected colonic tissue. This strain has been deposited according to the Budapest Treaty at Deutsche Sammlung von Mikroorganismen und Zellkulturen GmbH, Braunschweig, Germany, on October 24, 2000 with the accession number DSM 13801.

Strain F: *Bifidobacterium breve* 134 has good resistance to bile and low pH and exhibits fast growth in conventional laboratory media. This strain has been deposited according to the Budapest Treaty at Deutsche Sammlung von Mikroorganismen und Zellkulturen GmbH, Braunschweig, Germany, on October 24, 2000 with the accession number DSM 13802.

Strain G: *Bifidobacterium longum* 46 has good resistance to bile and oxygen. This strain has been deposited according to the Budapest Treaty at Deutsche Sammlung von Mikroorganismen und Zellkulturen GmbH, Braunschweig, Germany, on October 15, 2001 with the accession number DSM 14583.

Strain H: *Bifidobacterium longum* 51 has good adhesion to intestinal mucus and good resistance to oxygen. This strain has been deposited according to the Budapest Treaty at Deutsche Sammlung von Mikroorganismen und Zellkulturen GmbH, Braunschweig, Germany, on October 15, 2001 with the accession number DSM 14584.

The following strains I to L, which form a still further object of this invention, were isolated from faecal specimens from very old, healthy Finnish women and men. The strains were selected using the above indicated selection criteria.

Strain I: *Bifidobacterium adolescentis* 2C exhibits good adhesion-both to intestinal mucus and to whole intestinal mucosa. It has good acidification, growth and survival in oat-based media. The strain also has good resistance to oxygen. This strain has been deposited according to the Budapest Treaty at Deutsche Sammlung von Mikroorganismen und Zellkulturen GmbH, Braunschweig, Germany, on October 15, 2001 with the accession number DSM 14579.

Strain J: *Bifidobacterium longum* 3A exhibits good adhesion and has good resistance to bile and oxygen. This strain has been deposited according to the Budapest Treaty at Deutsche Sammlung von Mikroorganismen und Zellkulturen GmbH, Braunschweig, Germany, on October 15, 2001 with the accession number DSM 14580.

Strain K: *Bifidobacterium longum* 6 exhibits good adhesion and has good resistance to bile and oxygen. This strain has been deposited according to the Budapest Treaty at Deutsche Sammlung von Mikroorganismen und Zellkulturen GmbH, Braunschweig, Germany, on October 15, 2001 with the accession number DSM 14581.

Strain L: *Bifidobacterium adolescentis* 9J has good acidification, growth and survival in oat-based media. This strain has been deposited according to the Budapest Treaty at Deutsche Sammlung von Mikroorganismen und Zellkulturen GmbH, Braunschweig, Germany, on October 15, 2001 with the accession number DSM 14582.

All the above strains are non-spore forming Gram-positive anaerobes with rod or 'Y'-shaped cell morphology. Their shape and colony morphology on BL-agar is typical to bifidobacteria (round, smooth, brown). All the strains ferment ribose, glucose, fructose, galactose, sucrose, maltose, cellobiose, lactose, melibiose, sorbitol (A only weakly) and raffinose, but none of them ferment melezitose or inositol. The strains show mutual differences in the fermentation of arabinose and xylose [strain F (-), others (+)], mannose [A, E, G, H, J, K and L (-), F unknown, others (+)], trehalose [A, F, G, H, I, J, K and L (-), others (+)], starch [D, G, H, I, J, K (-), others (+)], inulin [A and E (+), others (-)], glycogen [D, G, H, I, J, K, L (-), others (+)] and mannitol [E, G, H, I, J, K, L (-), others (+)].

Further strains can be isolated in a similar way to contain all or some of the above-mentioned properties, for use in disease-specific functional foods and foods for the reduction of risk of diseases such as intestinal cancers.

The resulting strains can be specifically applied for the target populations, especially elderly people, to produce functional foods for such populations.

For instance, the strains *B. adolescentis* 5, *B. adolescentis* 15 and *B. longum* 56, isolated from healthy elderly Japanese men, where found to adhere to the intestinal mucosa of colon cancer patients.

Table 1 below shows advantageous properties of the *Bifidobacterium* strains of the invention as compared to the probiotic prior art strain *Bifidobacterium lactis* Bb12.

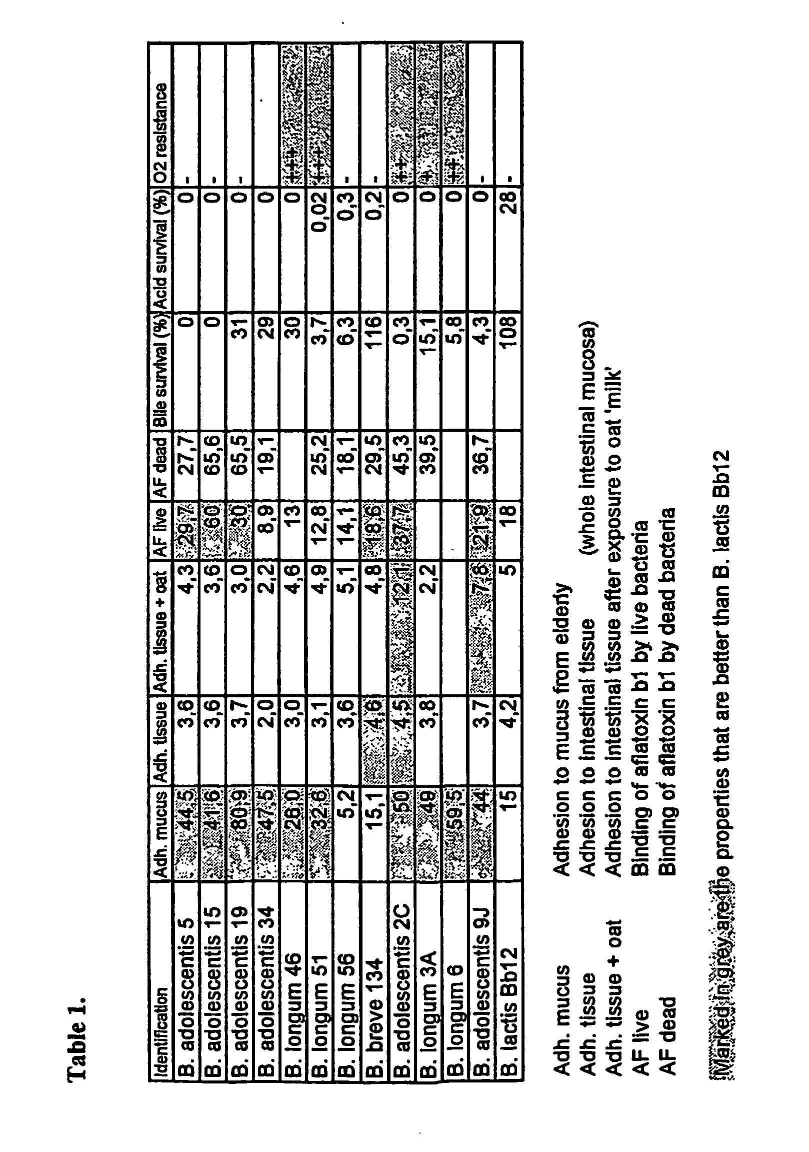

### EXPERIMENTAL

### Isolating bifidobacteria from faecal specimens

The collected faecal samples were kept at 4°C and analysed within 6 hours. Fecal bifidobacteria were isolated and identified using the methods reported by Mitsuoka [(1980) A color atlas of anaerobic bacteria. Sobunsya, Tokyo. pp. 341]. In brief, the fecal samples were homogenised, and a 10-fold serial dilution was prepared with an anaerobic buffer solution. Fifty microliters of appropriate dilutions were plated on the Blood Liver agar (BL agar; Nissui Seiyaku Co., Tokyo, Japan). The inoculum was incubated under anaerobic conditions at 37°C for 72h. The characteristic brown colonies formed on BL agar were randomly picked as potential bifidobacteria and further confirmed by Gram stain, cell morphology, absence of growth under aerobic conditions and spore formation. The strains were identified to species level based on their sugar fermentation pattern, as described by Mitsuoka [(1980) A color atlas of anaerobic bacteria. Sobunsya, Tokyo. pp. 341]. Isolated bifidobacteria were stored at -75°C in 50% glycerol until use.

### Screening for adhesion properties by in vitro adhesion assay using isolated intestinal mucus

Mucus was isolated from the faeces by extraction and dual ethanol precipitation [Kirjavainen et al. (1998) FEMS Microbiol. Lett. 167:185-189; Millerand & Hoskins (1981) Gastroenterology 81:759-765]. In short, faeces from healthy elderly volunteers were suspended in ice-cold PBS containing protease inhibitors and sodium azide. Fecal extracts were prepared by centrifuging the suspensions at 15000 x g at 4°C. Mucus was isolated from the fecal extract by dual ethanol precipitation. The pooled mucus was dissolved in HEPES (N-(2-hydroxyethyl)piperazine N-2(ethane sulfonic acid) buffered Hanks' balanced salt solution (HH; 10 mM HEPES; pH 7.4) at a concentration of 1 mg ml⁻¹. Any particulate material was removed from the suspension by centrifugation (2000 x g for 10 min).

The tested bacteria were grown in 1 ml of GAM broth (Nissui Seiyaku Co., Tokyo, Japan) from frozen stocks. To metabolically radiolabel the bacteria, 20 µl (*methyl-*1,2-³H) thymidine, (117 Ci mmol⁻¹) was added to the medium. After approximately 36 h of anaerobic growth at 37°C, the bacteria were harvested by centrifugation (2000 x g, 7 min), washed twice and resuspended in HH buffer. The absorbance at 600 nm was adjusted to 0.25 ± 0.01 in order to standardise the number of bacteria (approximately 10⁷ CFU ml⁻¹).

The adhesion assay was performed essentially as reported earlier [Kirjavainen et al. (1998) FEMS Microbiol. Lett. 167, 185-189; Ouwehand (1999) FEMS Microbiol. Lett. 172, 61-64]. In short, the intestinal mucus was passively immobilised on polystyrene microtitre plate wells by overnight incubation at 4°C. The wells were washed with HH buffer and the radioactively labelled bacteria were added. After 1.5 h incubation at 37°C, the wells were washed and the adhered bacteria were lysed with 1% SDS in 0.1 M NaOH at 60°C for 1h. The radioactivity of the lysed bacteria was assessed by liquid scintillation. Adhesion is expressed as the percentage of radioactivity recovered from the wells compared to radioactivity added to the wells.

### Screening for adhesion properties by ex vivo adhesion assay using resected intestinal tissue

Colonic tissue was resected from colon cancer patients by surgery. The diseased part of the resected tissue was used for adhesion studies to study disease-specificity and the healthy part for studying adhesion to healthy human mucosa. The resected material was collected on ice within 20 min after resection and processed immediately. The intestine was cut open and possible contents were removed. Subsequently, the tissue was gently washed in PBS containing 0.01% gelatin until no contents were visible. From the tissue 9 mm circular pieces (64 mm²) were stamped. The pieces were stored at -70°C in PBS with 40% glycerol until use.

For the adhesion to the human colonic tissue a method modified after Henriksson et al. [(1991) Appl. Environ. Microbiol. 57:499-502] was used. The tissue pieces were thawed and gently washed in HH. The pieces were then placed in wells of 24-well tissue culture plates, mucosal side up. To the wells 0.5 ml of radioactively labelled bacteria were added, to cover the tissue. The plates were incubated for 1 h in a 37°C water bath. Subsequently, each well was washed twice with 1 ml of PBS and the tissue pieces were fixed with 3% glutaraldehyde in PBS for 45 min at room temperature. The mucosa was then separated from the muscle tissue with a scalpel and digested overnight with 300 µl of 30% H₂O₂ and 150 µl of 70% perchloric acid in glass scintillation vials at 60-70°C. After digestion, 4 ml of scintillant was added and the radioactivity determined by liquid scintillation. The percentage adhesion was determined from the radioactivity in 0.5 ml of the original bacterial suspension as mentioned above for colonic mucus.

Selecting strains which, when binding intestinal toxins lose their adherence to the mucus Bacteria were grown in GAM broth (Nissui Seiyaku Co., Tokyo, Japan) overnight under anaerobic conditions. The bacteria were washed twice with phosphate buffered saline (PBS) and the concentration was adjusted to 1x10⁸ CFU/ml using the method described by Virta and co-workers [(1998) Appl. Environ. Microbiol. 2:515-519]. Aflatoxin B1 (5 µg/ml) in PBS was added, the mixture was incubated at 37°C for 4 h and supernatants analysed with HPLC as previously described [El-Nezami et al. (1996) Nutr. Today 31:41-42]. The adhesion assay to intestinal mucus was performed as described above, with aflatoxin B I-preincubated as well as non-treated bacterial suspensions.

### Influence of the food matrix on tissue adhesion

The influence of the food matrix on the adhesion of microorganisms has been investigated only marginally for milk. It was observed that milk significantly inhibited the adhesion of all tested probiotic microorganisms. Using the new whole intestinal mucosa model, the effect of cereal substrates, especially oats, on the adhesion of the bifidobacteria was investigated. In contrast to the observations for milk (Ouwehand et al. 2001, Int. J. Food Microbiol. 64:119-126), oat substrate was observed to more than double the adhesion to the whole intestinal mucosa (*ex vivo*) of selected *B. adolescentis* strains. Other tested strains were not affected in their (*ex vivo*) adhesion This suggests that a cereal substrate, especially oat substrate has a positive or no effect on the adhesion of probiotic bacteria, while milk has a strong negative effect on the adhesion of probiotics (Ouwehand et al. 2001 Int. J. Food Microbiol. 64:119-126).

The effect of ash, protein and fibre content was investigated using different mill fractionated cereals; oats, rye and barley. *Bifidobacterium* strains showed to grow in all fractions but the best was oats, and oats also increased the tissue adhesion.

### Selecting strains likely to survive the passage through the intestinal tract

Bacteria were grown in GAM broth (Nissui Seiyaku Co., Tokyo, Japan) overnight under anaerobic conditions. The bacteria were washed twice with phosphate buffered saline (PBS) and the concentration was adjusted to approximately 10⁸ CFU/ml by adjusting the absorbance at 600 nm to 0.5. The bacteria were centrifuged, resuspended in PBS, PBS+0.3% Oxgall or PBS at pH 2.5 and incubated for 60 min at 37°C. Viability in the different incubations was assessed by plate counting on BL-agar and compared to the PBS incubation.

Screening strains with the ability to grow on cereal oligosaccharides Flour fractions enriched in oat starch or oat β-glucan were produced. From these fractions and whole oat flour, a stable slurry was produced to obtain an oat-drink. Strains were inoculated in this drink, and their growth (determined as CFU/ml on BL-agar) and ability to acidify the substrate were assessed.

### Screening for the aerotolerance of the bacteria

Bacteria were grown in GAM broth (Nissui Seiyaku Co., Tokyo, Japan) overnight under anaerobic conditions. The bacteria were spread on BL-agar and incubated under strict anaerobic conditions (control), under aerobic conditions and under conditions with 3-5% oxygen. Possible growth on the latter two was compared to growth on the control.

### Screening for the most effective strains that inhibit the growth of common intestinal pathogens

Bacteria were grown in GAM broth (Nissui Seiyaku Co., Tokyo, Japan) overnight under anaerobic conditions. The bacteria were removed from the broth by centrifugation. The spent culture liquid was neutralised to pH 7.0 by the addition of 1M NaOH and filter-sterilised. Intestinal pathogens were grown overnight at 37° in an appropriate medium (Luria-Bertani-broth; LB-broth). Neutralised spent culture liquid was mixed in 1:1 ratio with double concentrated LB-broth and inoculated with intestinal pathogens. The growth of the pathogens was assessed by determining the absorbance at 600 nm at regular intervals. The results were compared to growth in fresh GAM broth mixed with double concentrated LB-broth as control.

### Screening for the most effective strains that inhibit the adhesion of common intestinal pathogens

Mucus was isolated from the faeces by extraction and dual ethanol precipitation as described above.

### In vitro adhesion assay

The tested bacteria were grown in 1 ml GAM broth (Nissui Seiyaku Co., Tokyo, Japan) from frozen stocks. After approximately 36 h of anaerobic growth at 37°C, the bacteria were harvested by centrifugation (2000 x g, 7 min), washed twice and resuspended in HH buffer.

The absorbance at 600 nm was adjusted to 0.25 ± 0.01 in order to standardise the number of bacteria (approximately 10⁷ CFU ml⁻¹).

The intestinal pathogens were grown in LB-broth and metabolically radiolabeled with 10 µl (*methyl-*1,2-³H) thymidine (117 Ci mmol⁻¹) in the medium. After overnight growth at 37°C, the bacteria were harvested by centrifugation (2000 x g, 7 min), washed twice and resuspended in HH buffer. The absorbance at 600 nm was adjusted to 0.25 ± 0.01 in order to standardise the number of bacteria (approximately 10⁷ CFU ml⁻¹).

The adhesion assay was performed essentially as reported earlier [Kirjavainen et al. (1998) FEMS Microbiol. Lett. 167, 185-189; Ouwehand et al. (1999) FEMS Microbiol. Lett 172, 61-64]. In short, the intestinal mucus was passively immobilised on polystyrene microtitre plate wells by overnight incubation at 4°C. The wells were washed with HH buffer and the bacteria to be assessed were added. After 1 h incubation at 37°C, the wells were washed and the radiolabelled intestinal pathogens were added and incubated again for 1h at 37°C. As a control, intestinal pathogens were also allowed to adhere to immobilised intestinal mucus not pre-treated with the test bacteria. The adhered bacteria were lysed with 1% SDS in 0.1 M NaOH at 60°C for 1h. The radioactivity of the lysed bacteria was assessed by liquid scintillation. The effect of the test bacteria on the adhesion of the intestinal pathogens was expressed as the percentage of radioactivity recovered from the wells pre-treated with test bacteria, compared to radioactivity recovered from the wells without test bacteria.

### Determining the absorption of aflatoxin B1 and other mycotoxins to viable and heat-killed strains

Bacteria were grown in GAM broth (Nissui Seiyaku Co., Tokyo, Japan) overnight under anaerobic conditions. The bacteria were washed twice with phosphate buffered saline (PBS) and the concentration was adjusted to 1x10¹⁰ CFU/ml using the method described by Virta and co-workers [(1998) Appl. Environ. Microbiol. 2:515-519]. Aflatoxin B1 (5 µg/ml) in PBS was added, the mixture was incubated at 37°C for 4 h and supernatants analysed with HPLC as previously described [E1-Nezami et al. (1996) Nutr. Today 31:41-42]. The tested bacteria were heat killed by boiling for 1h.

Determining the influence of the host microflora on their mucus-binding properties, using the specific target group's own microflora as a selection criterion Mucus was isolated as described above. The adhesion assay was modified from the description under the paragraph 'Screening for adhesion properties by *in vitro* adhesion assay...'. In short, intestinal mucus was immobilised on polystyrene microtitre plate wells by overnight incubation at 4°C. The wells were washed with HH. To the wells, 100 µl PBS, faecal extract, faecal bacteria or washed faecal bacteria were added and incubated for 1 h at 37°C. The wells were subsequently washed and 100 µl radiolabelled probiotic bacteria were added. After 1h incubation at 37°C, the wells were washed and the adhered bacteria were lysed with 1% SDS in 0.1 M NaOH. The radioactivity of the lysed bacteria was assessed by liquid scintillation. Adhesion is expressed as the percentage of radioactivity recovered from the wells compared to the the radioactivity added to the wells.

### Preparing of food products and dietary supplements

### Example 1. Cold Oat Meal with Bifidobacteria

One or more of the strains *Bifidobacterium adolescentis* 5, 15, 19, 34, 2C, 9J, *Bifidobacterium longum* 51, 56, 46, 3A, 6 or *Bifidobacterium breve* 134 were cultivated in a medium:

| | |
|---|---|
| Trypticase peptone | 8 g/l |
| Phytone peptone | 3 g/l |
| Yeast extract | 5 g/l |
| K₂HPO₄ | 2 g/l |
| KH₂PO₄ | 3 g/l |
| MgCl₂.6H₂O | 0.5 g/l |
| FeSO₄.7H₂O | 10 mg/l |
| Propionate | 50 mg/l |
| Glucose | 20 g/l |
| L-cysteine-HCl | 0.5 g/l |

Growth conditions: pH controlled: 6.5; Temp: 37°C; Incubation time: approx. 2 days (until consumption of NaOH, to neutralise the medium, has stopped); N₂-atmosphere.

The bacterial cells were separated and freeze-dried. The cold oat meal was produced by mixing the dry cereal and berry ingredients with the powder of *Bifidobacterium* strain or strains as follows:

| | |
|---|---|
| Instant oat flakes | 75 % |
| Dry berry or fruit powder (blackberries, strawberries, banana, apple) | 10 % |
| Sugar | 14 % |
| Freeze-dried strain or strains of bifidobacteria (containing 10¹²-10¹³ cells/g) | 0,05 % |

The product was immediately gas-packed in nitrogen atmosphere in one-serving aluminium foil packages. The size of one serving is 35 g and it contains at least 10⁸-10⁹ bifidobacterial cells per serving. One serving of a dry meal is mixed with 3/4-1 dl of cold or lukewarm milk.

### Example 2. Fermented oat material

The following ingredients were mixed in a container suitable for heat treatment:
9.5 % oat flour
2.6 % resistant starch or oat starch
3.0 % oligofructose
84.9 % water

The mixture, being first gelatinised by steam heating to reach the temperature of 131°C and further kept at that temperature for 2-3 minutes, was mechanically homogenised and finally cooled at approximately 37°C. The slurry obtained was then transferred into a fermentation tank, and inoculated with one of the strains, or with a mixture of strains of *Bifidobacterium* species selected from *Bifidobacterium adolescentis* 5, 15, 19, 34, 2C, 9J, *Bifidobacterium longum* 56, 51, 46, 3A, 6 or *Bifidobacterium breve* 134.

Freeze-dried strain or strains of bifidobacteria contained 10¹² - 10¹³ colony forming units per gram. The concentration of the bacteria added into the slurry was 0.05 - 0.1 % calculated from the weight of the batch. The fermentation took place at 37°C for 18-24 hours affording to fermented material, which was characterised as follows; pH 4.2- 4.8, colony forming units of bifidobacteria per gram: 10⁸ - 10⁹, a total acidity of about 0.2-0.4 weight-%, expressed as lactose acid content.

The fermented cereal material can be stored under 8°C up to 2 days before using, or pasteurised for long-term storage. This material is suitable for the preparation of fruit desserts, fresh cheese, yoghurts, beverages etc. Dosage, means of insertion and finishing treatment are determined by the desired product.

### Example 3. Fermented material with oat and rice

The following ingredients were mixed in a container suitable for heat treatment:

| | |
|---|---|
| 6 % | oat flour |
| 1.5% | oat starch |
| 3.0 % | rice flour |
| 1.5 % | isolated soy proteinate |
| 3.0 % | oligofructose |
| 1.0% | glucose |
| 1.0 % | casein hydrolysate |
| 83 % | water |

The mixture was treated as described in Example 2 to obtain a corresponding product.

## Claims

1. A method for screening probiotic strains of the genus *Bifidobacterium,* comprising isolating bifidobacteria from faecal specimens of healthy elderly human subjects, and screening for their age- and disease-specific properties by screening for their adhesion to intestinal mucus glycoproteins and to whole intestinal mucosa, screening for the ability of the strains to grow on cereal-based substrates, as well as screening for the aerotolerance of the strains and, optionally, subjecting the bacteria further to the steps of
a) selecting for strains which, when binding intestinal toxins lose their adherence to the mucus,
b) selecting for strains likely to survive the passage through the intestinal tract, and
c) selecting for the most effective strains that inhibit common intestinal pathogens, and isolating and identifying the strains showing an appropriate selection of the above-indicated properties.

2. The method according to claim 1, wherein the bifidobacteria are isolated from freshly voided faecal specimens of healthy very old human subjects.

3. The method according to claim 1, wherein the screening for age-specific properties is effected by subjecting the bacteria to a test using human intestinal mucus glycoproteins and their age-dependent samples, to select for specific bacteria adhering to the mucus of very old healthy subjects.

4. The method according to claim 1, wherein the screening for disease-specific properties is effected by subjecting the bacteria to a test using human intestinal mucus glycoproteins or whole intestinal mucosa from subjects with different intestinal dysfunctions.

5. The method according to claim 1, wherein the selection for strains which, when binding intestinal toxins lose their adherence to the mucus, is effected by placing the bacteria in a test using mycotoxin and/or bacterial toxin binding as a model for intestinal toxin binding.

6. The method according to claim 1, wherein the selection of strains likely to survive the passage through the intestinal tract is effected by testing their relative resistance to bile and low pH by assessing their viability with the help of flow cytometry.

7. The method according to claim 1, wherein the screening for the ability of the strains to grow on cereal-based substrates is effected by cultivating the strains on different substrates, and selecting strains being able to grow on substrates of interest.

8. The method according to claim 7, wherein the cereal-based substrates are oat-based substrates.

9. The method according to claim 8, wherein the oat-based substrates are oat carbohydrates.

10. The method according to claim 1, wherein the screening for the aerotolerance of the bacteria is effected by culturing the strains on blood liver agar plates in an atmosphere with reduced oxygen pressure.

11. The method according to claim 1, wherein the selection for strains inhibiting common intestinal pathogens is effected by subjecting the bacteria to competitive exclusion of model pathogens.

12. A biologically pure culture of a probiotic *Bifidobacterium* strain, selected from the strains *Bifidobacterium adolescentis* 2C, having the accession number DSM 14579, *Bifidobacterium longum* 3A, having the accession number DSM 14580, *Bifidobacterium longum* 6, having the accession number DSM 14581, *Bifidobacterium longum* 46, having the accession number DSM 14583 and *Bifidobacterium longum* 51, having the accession number DSM 14584.

13. Use of the probiotic *Bifidobacterium* strains as defined in claim 12 for producing functional or clinical food products or dietary supplements.

14. Use according to claim 13, wherein the functional or clinical food products are age-specific or disease-specific functional or clinical food products.

15. A functional or clinical food product, comprising at least one of the *Bifidobacterium* strains as defined in claim 12.

16. A dietary supplement, comprising at least one of the *Bifidobacterium* strains as defined in claim 12.

## Patentansprüche

1. Verfahren zum Screenen probiotischer Stämme der Gattung *Bifidobacterium,* umfassend das Isolieren von Bifidobakterien aus Stuhlproben gesunder älterer menschlicher Individuen, und das Screenen auf deren alters- und krankheitsspezifische Eigenschaften durch Screenen auf ihre Adhäsion an intestinale muköse Glycoproteine und an die gesamte intestinale Schleimhaut, Screenen auf die Fähigkeit der Stämme, auf auf Getreide basierenden Substraten zu wachsen, sowie das Screenen auf die Aerotoleranz der Stämme und, gegebenenfalls, des Weiteren das Unterziehen der Bakterien den Schritten des
(a) Auswählens von Stämmen, die, wenn sie an intestinale Toxine binden, ihre Adhärenz an den Schleim verlieren,
(b) Auswählens von Stämmen, die voraussichtlich die Passage durch den Intestinaltrakt überleben, und
(c) Auswählens der wirksamsten Stämme, die übliche intestinale Pathogene hemmen, und
das Isolieren und Identifizieren der Stämme, die eine geeignete Auswahl der obengenannten Eigenschaften aufweisen.

2. Verfahren nach Anspruch 1, wobei die Bifidobakterien aus frisch entleerten Stuhlproben gesunder, sehr alter menschlicher Individuen isoliert werden.

3. Verfahren nach Anspruch 1, wobei das Screenen auf altersspezifische Eigenschaften **dadurch** durchgeführt wird, dass die Bakterien einem Test unter Verwendung menschlicher intestinaler muköser Glycoproteine und deren altersabhängigen Proben unterzogen werden, um spezifische Bakterien auszuwählen, die an Schleim sehr alter gesunder Individuen anhaften.

4. Verfahren nach Anspruch 1, wobei das Screenen auf krankheitsspezifische Eigenschaften **dadurch** durchgeführt wird, dass die Bakterien einem Test unter Verwendung menschlicher intestinaler muköser Glycoproteine oder gesamter intestinaler Schleimhaut aus Individuen mit verschiedenen intestinalen Dysfunktionen unterzogen werden.

5. Verfahren nach Anspruch 1, wobei die Auswahl von Stämmen, die, wenn sie intestinale Toxine binden, ihre Adhärenz an den Schleim verlieren, **dadurch** durchgeführt wird, dass die Bakterien in einen Test eingebracht werden, der Binden an Mykotoxin und/oder bakterielles Toxin als Modell für Binden an intestinales Toxin verwendet.

6. Verfahren nach Anspruch 1, wobei die Auswahl der Stämme, die voraussichtlich die Passage durch den Intestinaltrakt überleben, **dadurch** durchgeführt wird, dass ihre relative Widerstandsfähigkeit gegenüber Gallenflüssigkeit und niedrigem pH-Wert durch Feststellen ihrer Lebensfähigkeit unter Verwendung von Durchflusscytometrie getestet wird.

7. Verfahren nach Anspruch 1, wobei das Screenen auf die Fähigkeit der Stämme, auf auf Getreide basierenden Substraten zu wachsen, durch Züchten der Stämme auf verschiedenen Substraten und Auswählen der Stämme durchgeführt wird, die in der Lage sind, auf einem Substrat von Interesse zu wachsen.

8. Verfahren nach Anspruch 7, wobei die auf Getreide basierenden Substrate auf Hafer basierende Substrate sind.

9. Verfahren nach Anspruch 8, wobei die auf Hafer basierenden Substrate Haferkohlenhydrate sind.

10. Verfahren nach Anspruch 1, wobei das Screenen auf die Aerotoleranz der Bakterien durch Züchten der Stämme auf Blut-Leber-Agar-Platten in einer Atmosphäre mit vermindertem Sauerstoffdruck durchgeführt wird.

11. Verfahren nach Anspruch 1, wobei die Auswahl von Stämmen, die übliche intestinale Pathogene hemmen, **dadurch** durchgeführt wird, dass die Bakterien einem kompetitiven Ausschluss von Modellpathogenen unterzogen werden.

12. Biologisch reine Kultur eines probiotischen *Bifidobacterium-*Stammes, ausgewählt aus den Stämmen *Bifidobacterium adolescentis* 2C mit der Zugangsnummer DSM 14579, *Bifidobacterium longum* 3A mit der Zugangsnummer DSM 14580, *Bifidobacterium longum* 6 mit der Zugangsnummer DSM 14581, *Bifidobacterium longum* 46 mit der Zugangsnummer DSM 14583 und *Bifidobacterium longum* 51 mit der Zugangsnummer DSM 14584.

13. Verwendung der wie in Anspruch 12 definierten probiotischen *Bifidobacterium-*Stämme für die Herstellung funktioneller oder klinischer Nahrungsprodukte oder Nahrungsergänzungsmittel.

14. Verwendung nach Anspruch 13, wobei die funktionellen oder klinischen Nahrungsprodukte altersspezifische oder krankheitsspezifische funktionelle oder klinische Nahrungsprodukte sind.

15. Funktionelles oder klinisches Nahrungsprodukt, umfassend mindestens einen der wie in Anspruch 12 definierten *Bifidobacterium-*Stämme.

16. Nahrungsergänzungsmittel, umfassend mindestens einen der wie in Anspruch 12 definierten *Bifidobacterium-*Stämme.

## Revendications

1. Procédé de criblage de souches probiotiques du genre *Bifidobacterium,* comprenant l'isolement de bifidobactéries à partir de spécimens fécaux de sujets humains âgés sains et le dépistage de leurs propriétés spécifiques à l'âge ou à une maladie par dépistage de leur adhérence aux glucoprotéines du mucus intestinal et à l'ensemble de la muqueuse intestinale, le dépistage de l'aptitude des souches à se développer sur des substrats à base de céréales, ainsi que le dépistage de l'aérotolérance des souches et éventuellement le fait de soumettre les bactéries en outre aux étapes consistant à
a) sélectionner des souches qui, quand elles fixent des toxines intestinales, perdent leur adhérence au mucus,
b) sélectionner des souches susceptibles de survivre au passage à travers l'appareil intestinal et
c) sélectionner les souches les plus efficaces qui inhibent des agents pathogènes intestinaux courants et isoler et identifier les souches présentant une sélection appropriée des propriétés indiquées ci-dessus.

2. Procédé selon la revendication 1, dans lequel les bifidobactéries sont isolées à partir de spécimens fécaux fraîchement évacués de sujets humains sains très vieux.

3. Procédé selon la revendication 1, dans lequel le dépistage des propriétés spécifiques à l'âge est effectué en soumettant les bactéries à un essai en utilisant des glucoprotéines du mucus intestinal et leurs échantillons dépendant de l'âge pour sélectionner des bactéries spécifiques qui adhèrent au mucus de sujets sains très vieux.

4. Procédé selon la revendication 1, dans lequel on effectue le dépistage de propriétés spécifiques à la maladie en soumettant les bactéries à un essai en utilisant des glucoprotéines humaines du mucus intestinal ou la totalité de la muqueuse intestinale de sujets atteints de différents dysfonctionnements intestinaux.

5. Procédé selon la revendication 1, dans lequel on effectue la sélection des souches qui, quand elles fixent des toxines intestinales, perdent leur adhérence au mucus, en plaçant les bactéries dans un essai en utilisant une liaison de mycotoxines et/ou de toxines bactériennes comme modèle pour la fixation de toxines intestinales.

6. Procédé selon la revendication 1, dans lequel on effectue la sélection de souches susceptibles de survivre au passage à travers l'appareil intestinal en testant leur résistance relative à la bile et à un pH bas en évaluant leur viabilité à l'aide d'une cytométrie de flux.

7. Procédé selon la revendication 1, dans lequel on effectue le dépistage de l'aptitude des souches à se développer sur des substrats à base de céréales en cultivant les souches sur différents substrats et en sélectionnant les souches capables de se développer sur des substrats présentant un intérêt.

8. Procédé selon la revendication 7, dans lequel les substrats à base de céréales sont des substrats à base d'avoine.

9. Procédé selon la revendication 8, dans lequel les substrats à base d'avoine sont des glucides d'avoine.

10. Procédé selon la revendication 1, dans lequel on effectue le dépistage de l'aérotolérance des bactéries en cultivant les souches sur des plaques de gélose et de sang du foie dans une atmosphère avec une pression réduite d'oxygène.

11. Procédé selon la revendication 1, dans lequel on effectue la sélection des souches inhibant des agents pathogènes intestinaux courants en soumettant les bactéries à une exclusion par compétition d'agents pathogènes servant de modèles.

12. Culture biologiquement pure d'une souche probiotique de *Bifidobacterium* choisie parmi les souches *Bifidobacterium adolescentis* 2C ayant le numéro d'accès DSM 14579, *Bifidobacterium longum* 3A ayant le numéro d'accès DSM 14580, *Bifidobacterium longum* 6 ayant le numéro d'accès DSM 14581, *Bifidobacterium longum* 46 ayant le numéro d'accès DSM 14583 et *Bifidobacterium longum* 51 ayant le numéro d'accès DSM 14584.

13. Utilisation des souches probiotiques de *Bifidobacterium* selon la revendication 12 pour produire des produits alimentaires fonctionnels ou cliniques ou des compléments alimentaires.

14. Utilisation selon la revendication 13, dans laquelle les produits alimentaires fonctionnels ou cliniques sont des produits alimentaires fonctionnels ou cliniques spécifiques à l'âge ou spécifiques à une maladie.

15. Produit alimentaire fonctionnel ou clinique comprenant au moins l'une des souches de *Bifidobacterium* telles que définies dans la revendication 12.

16. Complément alimentaire comprenant au moins l'une des souches de *Bifidobacterium* telles que définies dans la revendication 12.
